# EUROPEAN PATENT APPLICATION

(11) **EP 3 872 489 A1**
(43) Date of publication of application: **01.09.2021**
(21) Application number: 20159404.1
(22) Date of filing: 25.02.2020
(51) Int. Cl.: G01N 33/497, G01N 33/53

(54) **SENSING APPARATUS**

(71) Applicant: Nokia Technologies Oy, 02610 Espoo (FI)
(72) Inventor: KIM, John D., Bridgewater, NJ 08807 (US); SHAH, Shreyas, Dayton, NJ 08810 (US)
(74) Representative: Swindell & Pearson Limited

(57) **Abstract**

An apparatus for sensing, the apparatus comprising: a plurality of genetically modified sensors and a transducer array. The plurality of genetically modified sensors comprise a plurality of subsets of sensors wherein the sensors are genetically modified so as to provide an output in response to one or more chemicals. The plurality of genetically modified sensors are genetically modified in a non-correlated manner so that different subsets of sensors are configured to provide a different affinity to different chemicals. The transducer array, comprises a plurality of pixels, wherein different subsets of the plurality of genetically modified sensors are coupled to different pixels so as to cause the pixels to provide outputs dependent upon the genetic modification of the subset of sensors coupled to the pixel. Detection of one or more chemicals by one or more of the genetically modified sensors causes an identifiable output signal to be provided by the transducer array.

## Description

### TECHNOLOGICAL FIELD

Embodiments of the present disclosure relate to a sensing apparatus. Some relate to sensing apparatus which can detect a plurality of chemicals so as to provide an artificial nose or tongue.

### BACKGROUND

Sensors for detecting chemicals or groups of chemicals are known. However, it is difficult to create sensors that can reliably detect different chemicals so as to provide an artificial nose or other similar apparatus.

### BRIEF SUMMARY

According to various, but not necessarily all, examples of the disclosure there may be provided an apparatus comprising: a plurality of genetically modified sensors comprising a plurality of subsets of sensors wherein the sensors are genetically modified so as to provide an output in response to one or more chemicals and wherein the plurality of genetically modified sensors are genetically modified in a non-correlated manner so that different subsets of sensors are configured to provide a different affinity to different chemicals; and a transducer array, comprising a plurality of pixels, wherein different subsets of the plurality of genetically modified sensors are coupled to different pixels so as to cause the pixels to provide outputs dependent upon the genetic modification of the subset of sensors coupled to the pixel, wherein detection of one or more chemicals by one or more of the genetically modified sensors causes an identifiable output signal to be provided by the transducer array.

The genetically modified sensors within a subset may be homogenous.

The identifiable output signal from the transducer array may comprise a combination of outputs from a plurality of different pixels.

The genetically modified sensors may be adsorbed to the pixels of the transducer array.

The transducer array may comprise at least one of mass sensitive transducers, light sensitive transducers, electrically sensitive transducers, heat sensitive transducers.

The genetically modified sensors may comprise at least one of viral particles, desiccation tolerant cells, synthetic peptides, randomized DNA, proteins and receptors.

The genetically modified sensors may be genetically modified to sense chemicals in at least one of, gas phase, liquid phase.

The genetically modified sensors may be genetically modified in a random or pseudo random manner.

The apparatus may comprise means for providing the identifiable output signal of the transducer array to an artificial intelligence module to enable the artificial intelligence module to classify the identifiable output signal.

Classifying the identifiable output signal may comprise one or more of: determining an identity of a chemical, determining a class of chemicals, determining concentration of a chemical.

The artificial intelligence module may be configured to use a pattern recognition algorithm to classify the identifiable output signal.

According to various, but not necessarily all, examples of the disclosure there may be provided an apparatus comprising means for: receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and wherein the identifiable output signal provides an indication of the presence of one or more chemicals detected by a plurality of genetically modified sensors coupled to the transducer array; and using artificial intelligence to classify the identifiable output signal by determining any one or more of: an identity of a chemical, a class of chemicals, concentration of a chemical.

Training data obtained by exposing the transducer array and plurality of genetically modified sensors to one or more known chemicals may be used to classify the identifiable output signal from the transducer array.

A pattern recognition algorithm may be used to classify the identifiable output signal from the transducer array.

According to various, but not necessarily all, examples of the disclosure there may be provided a method comprising: receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and wherein the identifiable output signal provides an indication of the presence of one or more chemicals detected by a plurality of genetically modified sensors coupled to the transducer array; and using artificial intelligence to classify the identifiable output signal by determining any one or more of: an identity of a chemical, a class of chemicals, concentration of a chemical.

According to various, but not necessarily all, examples of the disclosure there may be provided a computer program comprising computer program instructions that, when executed by processing circuitry, cause: receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and wherein the identifiable output signal provides an indication of the presence of one or more chemicals detected by a plurality of genetically modified sensors coupled to the transducer array; and using artificial intelligence to classify the identifiable output signal by determining any one or more of: an identity of a chemical, a class of chemicals, concentration of a chemical.

According to various, but not necessarily all, examples of the disclosure there may be provided an apparatus comprising processing circuitry; and memory circuitry including computer program code, the memory circuitry and the computer program code configured to, with the processing circuitry, cause the apparatus to: receive an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and wherein the identifiable output signal provides an indication of the presence of one or more chemicals detected by a plurality of genetically modified sensors coupled to the transducer array; and use artificial intelligence to classify the identifiable output signal by determining any one or more of: an identity of a chemical, a class of chemicals, concentration of a chemical.

According to various, but not necessarily all, examples of the disclosure there may be provided a system comprising a sensing apparatus and a processing apparatus; wherein the processing apparatus comprises: a plurality of genetically modified sensors comprising a plurality of subsets of sensors wherein the sensors are genetically modified so as to provide an output in response to one or more chemicals and wherein the plurality of genetically modified sensors are genetically modified in a non-correlated manner so that different subsets of sensors are configured to provide a different affinity to different chemicals; and a transducer array, comprising a plurality of pixels, wherein different subsets of the plurality of genetically modified sensors are coupled to different pixels so as to cause the pixels to provide outputs dependent upon the genetic modification of the subset of sensors coupled to the pixel, wherein detection of one or more chemicals by one or more of the genetically modified sensors causes an identifiable output signal to be provided by the transducer array; and the processing apparatus comprises means for: receiving the identifiable output signal from the transducer array; and using artificial intelligence to classify the identifiable output signal by determining any one or more of: an identity of a chemical, a class of chemicals, concentration of a chemical.

### BRIEF DESCRIPTION

Some examples will now be described with reference to the accompanying drawings in which:
Fig. 1 illustrates an apparatus;
Figs 2A to 2C illustrate an apparatus an output signal of an apparatus;
Figs. 3A to 3C illustrate an apparatus and an output signal of an apparatus:
Figs. 4A and 4B illustrate an apparatus;
Figs. 5A to 5E illustrate assembly of an apparatus;
Fig. 6 illustrates a method; and
Fig. 7 illustrates an apparatus.

### DETAILED DESCRIPTION

Examples of the disclosure relate to an apparatus 100 for sensing. The apparatus 100 uses genetically modified sensors 103 to enable a wide range of chemicals 115 to be sensed and identified.

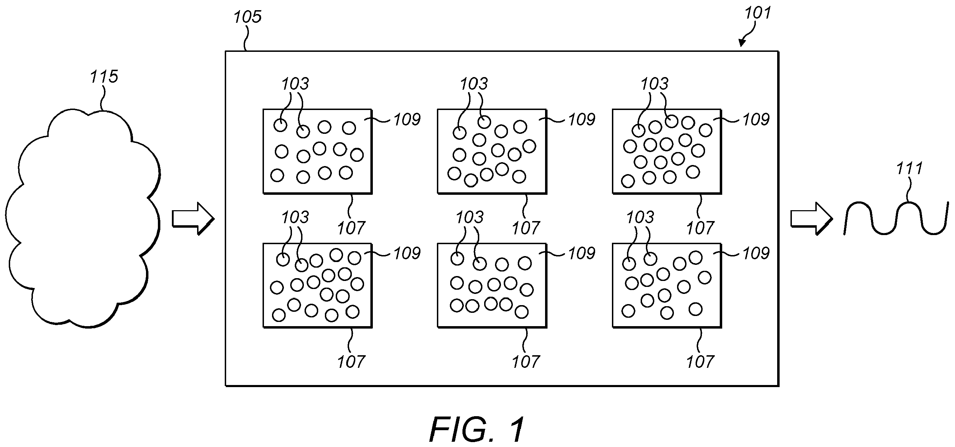

Fig. 1 schematically illustrates an example apparatus 101. The apparatus 101 comprises a plurality of genetically modified sensors 103 and a transducer array 105.

The genetically modified sensors 103 are genetically modified so as to provide an output in response to one or more chemicals 115. The genetically modified sensors 103 may comprise any biological material that can be genetically modified such that an output is provided when the sensors 103 detect one or more chemicals 115. The genetically modified sensors 103 comprise at least one of viral particles, desiccation tolerant cells, synthetic peptides, randomized DNA, proteins and receptors or any other suitable biological material. The genetically modified sensors 103 may be modified to sense chemicals in a gas phase and/or a liquid phase.

The plurality of genetically modified sensors 103 comprise a plurality of subsets 109 of sensors 103. The subsets 109 of sensors 103 are grouped so that sensors 103 within a subset 109 are genetically modified to respond to the same chemicals. The sensors 103 within a subset 109 may be homogenous. The sensors 103 within a subset 109 may be genetically modified in the same way so that they respond to the same chemicals 115 or provide the same affinity to the same types of chemicals 115. In some examples the sensors 103 within a subset 109 may all be the same.

In some examples there can be some variation between sensors 103 within a subset. For instance, in some examples a subset could comprise a mixture of two or more homogenous populations of sensors 103.

The plurality of different subsets 109 may be configured so that different subsets 109 comprise different types of sensors 103. This means that a first subset 109 comprises a different type of sensors 103 to a second subset 109. The different types of sensors 103 within different subsets 109 may be genetically modified in different ways so that they respond to different chemicals 115 or provide different affinities to different types of chemicals. In some examples each of the subsets 109 may comprise a different type of sensor 103 so that none of the subsets 109 are the same.

The plurality of genetically modified sensors 103 are genetically modified in a non-correlated manner. The non-correlated manner means that the genetic modification of a first subset 109 of sensors 103 is independent of the genetic modification of a second, different subset 109 of sensors 103. In some examples the non-correlated genetic modification may be random or pseudo random.

In some examples the non-correlated genetic modification could relate to a part of the genetic modification. For instance, in some examples the region where random nucleotides can be introduced into the sensor 103 could be limited. This may ensure that the sensors 103 retain some corresponding characteristics but can have other, non-correlated characteristics.

The non-correlated genetic modification may be used to create a library of genetically modified sensors 103. In some examples the sensors 103 used within the apparatus 101 may be selected from the library of genetically modified sensors 103. The sensors 103 may be selected randomly, or pseudo-randomly, from the library of sensors 103. The non-correlated genetic modification ensures that the different subsets 109 of sensors 103 are configured to provide a different affinity to chemicals 115 or types of chemicals. This enables the different subsets 109 of sensors 103 to provide different outputs in response to different chemicals 115 or types of chemicals. The different outputs provided by the different subsets 109 would be independent of each of other.

The apparatus 101 also comprises a transducer array 105. The transducer array 105 comprises a plurality of pixels 107 where each pixel 107 comprises one or more transducers. Different subsets 109 of the plurality of genetically modified sensors 103 are coupled to different pixels 107.

The genetically modified sensors 103 are coupled to the pixels 107 via any suitable means. The genetically modified sensors 103 are coupled to pixels 107 so that the output signal of the pixel 107 is dependent upon whether or not the sensors 103 have detected a chemical 115. In some examples the genetically modified sensors 103 may be adsorbed to the pixels 107. The adsorption could be chemical adsorption or physical adsorption. The chemical bond formed in a chemical adsorption process could be a covalent bond, a partially covalent bond or any other suitable type of bond. The type of coupling that is used to couple sensors 103 to the pixels 107 may be dependent upon the types of sensors 103 that are used and the types of transducers that are used within the pixels 107.

The transducers within the transducer array 105 may comprise any means which are configured to provide an output signal in response to the genetically modified sensors 103 coupled to the pixel 107 sensing one or more chemicals 115. In some examples the transducers could comprise mass sensitive transducers, light sensitive transducers, electrically sensitive transducers, heat sensitive transducers or any other suitable types of transducer.

In the example of Fig. 1 the pixels 107 are arranged in a regular rectangular array. It is to be appreciated that other arrangements of the pixels 107 could be used in other examples of the disclosure. In the schematic example of Fig. 1 six pixels 107 of the transducer array 105 are shown. It is to be appreciated that in other examples of the disclosure there could be more than six pixels 107. In some examples the number of pixels 107 within the array 105 could comprise tens or hundreds or thousands of pixels. The output provided by each of the pixels 107 is dependent upon the chemicals that are sensed by the sensors 103 coupled to the pixels 107. The output provided by each of the pixels 107 is therefore dependent upon the genetic modification of the subset 109 of sensors 103 coupled to the pixel 107.

The transducer array 105 is configured to provide an output signal that is dependent upon one or more chemicals 115 being detected by the genetically modified sensors 103. As different pixels 107 have different types of genetically modified sensors 103 coupled to them this causes different pixels 107 to provide different output signals. The different output signals from the pixels 107 within the transducer array 105 may be combined so as to provide an identifiable output signal 111.

The identifiable output signal 111 comprises information that enable one or more chemicals or types of chemicals to be identified. The identifiable output signal 111 can comprise information that enables a plurality of different chemicals to be identified. In some examples the identifiable output signal 111 can comprise information that enables a concentration of the chemicals or types of chemicals to be determined.

The identifiable output signal 111 from the apparatus 101 may be provided to an artificial intelligence module or any other suitable processor or controller so as to enable the identifiable output signal 111 to be classified. The artificial intelligence module may be configured to use a pattern recognition algorithm to classify the identifiable output signal 111. Classifying the output signal comprises one or more of: determining an identity of a chemical, determining a class of chemicals, determining concentration of a chemical.

The example apparatus 101 therefore provides an apparatus 101 for sensing chemicals 115. As the plurality of sensors 103 are genetically modified in a non-correlated manner this enables a large variation in the sensing capabilities of the plurality of sensors 103. By arranging these genetically modified sensors 103 into a plurality of non-correlated subsets 109 they can be coupled to a transducer array 105 so as to provide an output that can be classified using artificial intelligence. This enables the apparatus 101 to be used to detect different types and/or concentrations of different chemicals 115. This may enable the apparatus 101 to be used as an artificial nose or artificial tongue or other similar device.

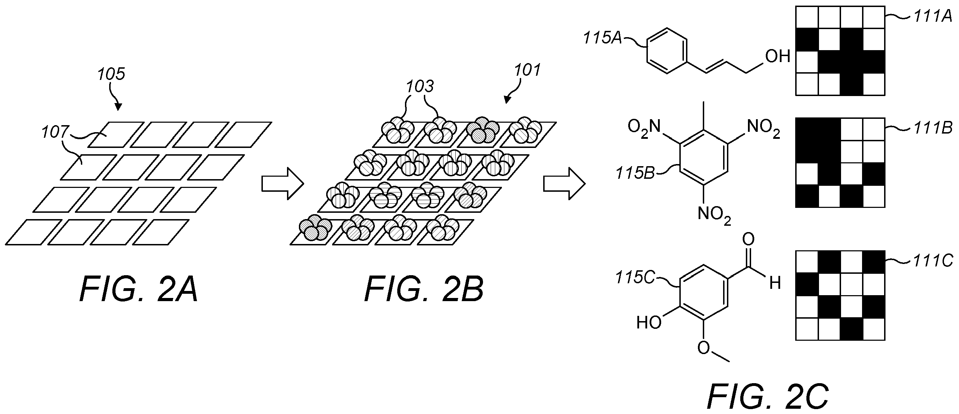

Figs. 2A to 2C schematically illustrate an apparatus 101 and example output signals 111 of the apparatus 101. In this example the apparatus 101 is configured as a general purpose apparatus 101 and can provide identifiable output signals 111 in response to different types of chemicals 115.

Fig. 2A schematically shows a transducer array 105 comprising sixteen pixels 107. The pixels 107 are arranged in an array comprising four rows and four columns. Other numbers of pixels 107 and arrangements of the pixels 107 could be used in other examples of the disclosure.

Each of the pixels 107 comprises one or more transducers. The transducers could comprise quartz crystal microbalance (QCM) transducers. The QCM transducer may be configured to detect a change in mass of the subset of sensors 109 coupled to the QCM transducer by measuring a change in frequency of a quartz crystal resonator. Other types of transducer could be used in other examples of the disclosure.

In Fig. 2B the genetically modified sensors 103 are coupled to the transducer array 105 to produce a sensing apparatus 101. In the schematic example of Fig. 2B only one sensor 103 is shown coupled to each pixel 107 for clarity. It is to be appreciated that a subset 109 comprising a plurality of sensors 103 would be coupled to each pixel 107 as described above. In this example each of the different types of sensors 103 used are created using random genetic modification. This leads to a general purpose sensing apparatus 101 which can differentiate between different types of chemicals.

Fig. 2C shows example output signals 111A, 111B, 111C that may be provided by the apparatus 101 in response to different chemicals 115A, 115B, 115C.

In this example the output signals 111 A, 111B, 111C comprise a matrix where each element of the matrix corresponds to a pixel 107 within the transducer array 105. The matrices comprise four rows and four columns corresponding to the transducer array 105. Each of the elements in the matrices provide an indication of whether or not the sensors 103 coupled to the corresponding pixel 107 have sensed the chemical 115A, 115B, 115C that the apparatus 101 has been exposed to. In the example shown in Fig. 2C the entry in matrix shows either a black element or a white element. The black element indicates that the sensor 103 has responded to the chemical 115 and the white element indicate that the sensor 103 has not responded to the chemical 115. This creates a matrix barcode output. Other types of output could be used in other examples of the disclosure.

In the example shown in Fig. 2C the apparatus 101 is exposed to three different types of aromatic chemicals. The first chemical 115A comprises a benzene ring with an alkene and primary alcohol. The second chemical 115B comprises trinitrotoluene. The third chemical 115C comprises a phenol-based chemical comprising a benzene ring, a primary alcohol, an ether and an aldehyde. Each of the chemicals 115A, 115B, 115C are sensed by different combinations of subsets 109 of the plurality of genetically modified sensors 103.

In the example shown in Fig. 2C the first chemical 115A is sensed by six of the subsets 109 of sensors 103 as shown in the output signal 111A. The second chemical 115B is sensed by eight of the subsets 109 sensors 103 as shown in the output signal 111B. Some of the sensors 103 that sense the first chemical 115A also sense the second chemical 115B while some of the sensors 103 that sense the first chemical 115A do not sense the second chemical 115B. Fig. 2C also shows that some of the sensors 103 that sense the second chemical 115B do not sense the first chemical 115A. This enables the second output signal 111B to be distinguished from the first output signal 111A. Similarly, the third chemical 115C is sensed by six of the subsets 109 of sensors 103 but in a different combination to the subsets 109 of sensors 103 that sense either the first chemical 115A or the second chemical 115B.

The different combinations of subsets 109 sensors 103 that sense the different chemicals 115A, 115B, 115C leads to different outputs being provided by different pixels of the transducer array 105. As shown in Fig. 2C different output matrices are given in response to each of the different chemicals 115A, 115B, 115C. The different output matrices can then be provided to an artificial intelligence module to enable the different chemicals 115A, 115B, 115C to be classified. The different chemicals 115A, 115B, 115C could be classified using a pattern recognition algorithm or any other suitable process.

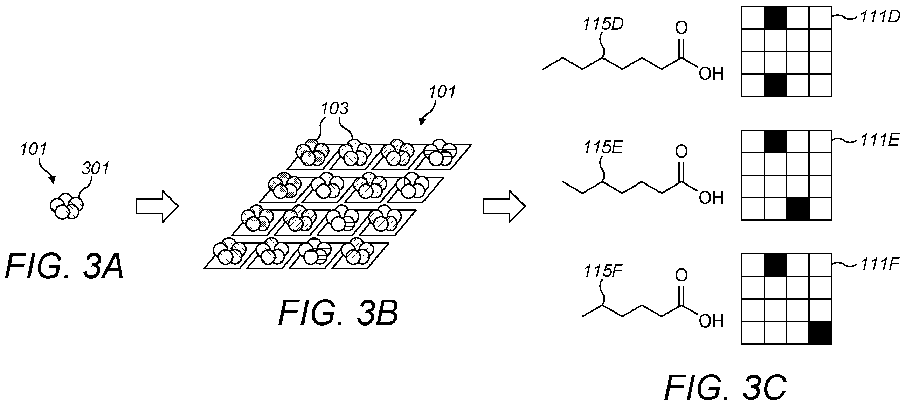

Figs. 3A to 3C schematically illustrate another apparatus 101 and example output signals 111 of the apparatus 101. In this example the apparatus 101 is configured as an application specific apparatus 101 and can provide identifiable output signals 111 in response to different variants of the same type of chemical 115.

Fig. 3A shows a first type of sensor 103. In this example the sensor 103 comprises a viral particle 301. The viral particle 301 may be selected based on its interaction with a type of chemical 115 that is to be sensed. In this example the genetic modification of the viral particle 301 may be restricted by limiting the region in which random nucleotides can be introduces on a viral gene. This allows for partially-random genetic modification. The genetically modified viral particles will retain some corresponding characteristics but can have other, non-correlated characteristics. The sensors 103 created using the partially-random process will be able to sense the same type of chemical 115 but will have different affinities to any variations of the chemical 115.

In Fig. 3B the genetically modified sensors 103 derived from the viral particle 301 are coupled to the transducer array 105 to produce a sensing apparatus 101. In the schematic example of Fig. 3B only one sensor 103 is shown coupled to each pixel 107 for clarity. It is to be appreciated that a subset 109 comprising a plurality of sensors 103 could be coupled to each pixel 107 as described above.

Fig. 3C shows example output signals 111D, 111E, 111F that may be provided by the apparatus 101 in response to different chemicals 115D, 115E, 115F.

In this example the output signals 111D, 111E, 111F comprise a matrix, as described above, where each element of the matrix corresponds to a pixel 107 within the transducer array 105.

In the example shown in Fig. 3C the apparatus 101 is exposed to three different variations of a type of chemical. In the example of Fig. 3C carboxylic acids with different chain lengths are used.

Each of the chemicals 115D, 115E, 115F are sensed by different combinations of subsets 109 of the plurality of genetically modified sensors 103. However there is some overlap for each of these combinations because the common features of the chemicals 115D, 115E, 115F could be detected by a common subset 109 of sensors 103. For instance, in this example the carboxyl group could be detected by the same subsets 109 of sensors 103.

In the example shown in Fig. 3C each of the carboxylic acids 115D, 115E, 115F is sensed by two of the subsets 109 of sensors 103. There is a first subset 109 of sensors 103 which is active for each of the carboxylic acids 115D, 115E, 115F. In the example of Fig. 3C this is shown as the matrix element in the first row, second column. This provides an indication that there is a relationship between the different chemicals 115D, 115E, 115F. In this example it indicates that they are all carboxylic acids.

The second subset 109 of sensors 103 that is active for each of the carboxylic acids 115D, 115E, 115F is different. In the example of Fig. 3C this is shown by different elements in the fourth row being active. This provides an indication that there is a variation in the different chemicals 115D, 115E, 115F. In this example it provides an indication of the different chain lengths.

The different output matrices can then be provided to an artificial intelligence module to enable the different chemicals 115D, 115E, 115F to be classified. The artificial intelligence module may recognize that the chemicals 115D, 115E, 115F are variants of each other by analysing the common active elements within the matrix. The artificial intelligence module may recognize the variations in the chemicals 115D, 115E, 115F by analysing the differences in the active elements in the matrices.

In the example of Figs. 2A to 3C the elements of the matrices are shown as either black or white. That is the pixels of the transducer array 105 have a binary output which indicates either that a chemical 115 has been sensed or has not been sensed. In other examples the output of the pixels 107 of the transducer array 105 could be analogue and/or could have more than two states. For instance, the intensity of the output provided by a pixel 107 of the transducer array 105 could provide an indication of the concentration of a chemical 115. In such examples the elements in the matrices could have varying shades of grey corresponding to the intensity of the output signal from the pixels 107.

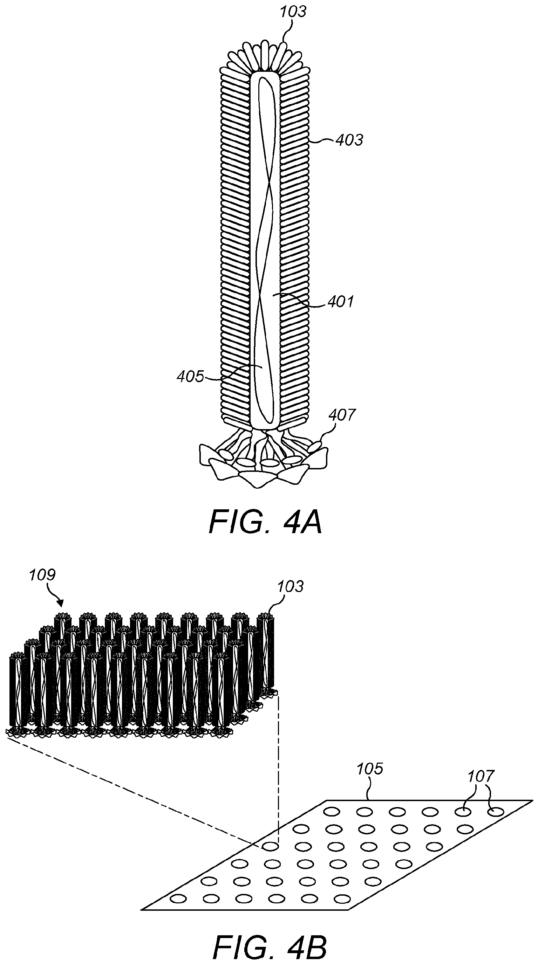

Figs. 4A and 4B illustrate a sensor 103 and an apparatus 101 comprising a plurality of sensors 103.

In the example of Fig. 4A the sensor 103 comprises an M13 filamentous bacteriophage 401. Other types of bio material could be used in other examples of the disclosure. For example, other types of viral particles could be used or other types material such as desiccation tolerant cells, synthetic peptides, randomized DNA, proteins and receptors could be used.

The filamentous bacteriophage 401 has a length of one to two micrometers long and several nanometers in diameter and comprises a DNA core 405. In this example the use of the filamentous structures may ensure that the plurality of sensors 103 provide a large surface area to volume ratio. This helps to increase the efficiency with which the chemicals 115 can be sensed.

A major coat protein 403 is provided around the filamentous bacteriophage 401. The major coat protein 403 is provided on the surface of the filamentous bacteriophage 401 and can be modified so as to respond to different chemicals 115. The major coat protein 403 may be modified by non-correlated genetic modification so that different filamentous bacteriophages 401 used within an apparatus 101 respond to different chemicals 115 in a non-correlated manner.

The major coat protein 403 may also provide an easily detected output in response to one or more chemicals 115. The output could be a change in mass of the sensor, a change in optical properties, a change in electrical properties or a change of any other suitable properties of the sensor 103.

A minor coat protein 407 is provided at an end of the filamentous bacteriophage 401. The minor coat protein 407 may be modified so as to enable the filamentous bacteriophage 401 to be coupled to a transducer in a transducer array 105. The minor coat protein 407 may enable the sensor 103 to be chemically bonded or physically bonded to a transducer in a transducer array 105.

Fig. 4B shows how a subset 109 of sensors 103 comprising filamentous bacteriophages 401 can be coupled to a pixel 107 in a transducer array 105. In the example of Fig. 4B each of the filamentous bacteriophages 401 in the subset 109 are the same so that a homogenous subset 109 of sensors 103 is provided. Each of the filamentous bacteriophages 401 have been genetically modified in the same way so that they sense the same chemicals 115 or types of chemicals. The homogenous subset of sensors 103 are then coupled to a pixel 107 within the transducer array 105.

In Fig. 4B only one subset of sensors 103 is shown. However, it is to be appreciated that a subset of sensors 103 would be provided for each of the pixels 107 in the transducer array 105. Each of the subsets 109 of sensors 103 would be genetically modified in a different way so that the different subsets 109 of sensors 103 can sense different chemicals 115.

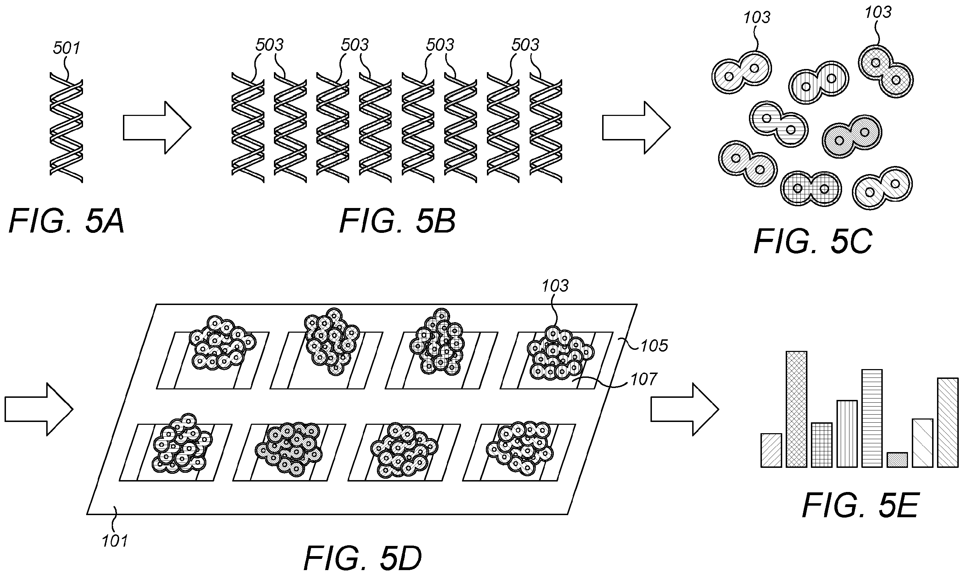

Figs. 5A to 5E schematically illustrate an assembly of an apparatus 101.

Fig. 5A shows a gene 501 that can encode sensors 103. The gene 501 can be modified to create a plurality of non-correlated gene variants 503 as shown in Fig. 5B. The non-correlated gene variants 503 may be random or pseudo random or partially random.

In Fig. 5C the gene variants 503 are then introduced into viral particles to create genetically modified sensors 103. In other examples the gene variants 503 may be introduced into desiccation tolerant cells or any other suitable type of material in order to create the genetically modified sensors 103.

The genetically modified sensors 103 are then coupled to a transducer array 105, to create an apparatus 101 as shown in Fig. 5D. The genetically modified sensors 103 are coupled to the transducer array 105 in subsets 109 so that different subsets 109 are coupled to different pixels of the transducer array 105.

The genetically modified sensors 103 could be coupled to the transducer array 105 using any suitable coupling. The coupling may comprise chemical bonding. The type of chemical bonds that are used may depend on the material used for the sensors 103 and or the material used for the transducers within the transducer array 105. For example, where the transducer array 105 comprises gold components the genetically modified sensors 103 could comprise a thiol group which will bond well to the gold. Other compounds or elements could be used in other examples of the disclosure.

In this example the transducer array 105 comprise an array of field effect transducers (FETs). Other types of transducer could be used in other examples of the disclosure. The FETs may detect a change in one or more electrical properties of the genetically modified sensors 103 when the genetically modified sensors 103 detect one or more chemicals 115.

The apparatus 101 provides an output, as shown in Fig. 5E, which is a combination of the response of the different pixels 107 within the transducer array 105. In the example of Fig. 5E the output comprises a histogram. The histogram may show the intensity of the outputs provided by the different pixels within the transducer array 105. These different intensities could provide a characteristic output that can be used to identify the chemical 115 that has been detected.

In the examples described above the output signals 111 may be provided from the apparatus 101 to an artificial intelligence module. The artificial intelligence module may then process the received output signal 111 so as to classify the chemicals 115 sensed by the apparatus 101.

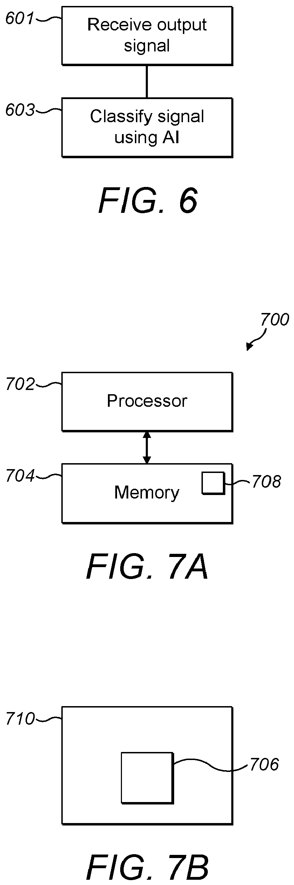

Fig. 6 illustrates a method that could be performed by an artificial intelligence module in examples of the disclosure.

At block 601 the method comprises receiving an identifiable output signal 111 from a transducer array 105. The transducer array 105 comprises a plurality of pixels 107 with a different subset 109 of genetically modified sensors 103 coupled to different pixels 107 within the transducer array 105. The sensors 103 have been genetically modified in a non-correlated manner so that different subsets 109 of sensors 103 are configured to provide a different affinity to different chemicals 115. The identifiable output signal 111 therefore provides an indication of the presence of one or more chemicals 115 detected by a plurality of genetically modified sensors 103 coupled to the transducer array 105.

At block 603 the method comprises using artificial intelligence to classify the identifiable output signal by determining any one or more of : an identity of a chemical, a class of chemicals, concentration of a chemical.

In some examples a pattern recognition algorithm could be used to classify the output signal 111 from the transducer array 105. For instance, where the output signal comprises a QR style output as shown in Figs 2C and 3C a pattern recognition algorithm could be used to identify which elements of the matrix show a response to a chemical 115.

In some examples the artificial intelligence may use training data to classify the identifiable output signal 111 from the transducer array 105. The training data could be obtained by exposing the transducer array 105 and plurality of genetically modified sensors 103 to one or more known chemicals. The training data could be obtained using different chemicals, different types of chemicals and/or different concentrations of chemicals and/or different conditions. For each chemical at different concentrations and conditions the output signal can be obtained and analysed to identify unique features within the output signal. The unique features could then be used to identify the chemical.

When the apparatus 101 is exposed to an unknown chemical 115 the obtained output signal 111 could be deconvoluted and compared to the training data so as to identify possibly matches to the unknown chemical.

The artificial intelligence module may use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines may be used for supervised learning. A Bayesian network is a directed acyclic graph that represents the conditional independence of a number of random variables.

Fig. 7A illustrates an example of a controller 700. The controller 700 could be provided within an artificial intelligence module. Implementation of a controller 700 may be as controller circuitry. The controller 700 may be implemented in hardware alone, have certain aspects in software including firmware alone or can be a combination of hardware and software (including firmware).

As illustrated in Fig. 7A the controller 700 may be implemented using instructions that enable hardware functionality, for example, by using executable instructions of a computer program 706 in a general-purpose or special-purpose processor 702 that may be stored on a computer readable storage medium (disk, memory etc) to be executed by such a processor 702.

The processor 702 is configured to read from and write to the memory 704. The processor 702 may also comprise an output interface via which data and/or commands are output by the processor 702 and an input interface via which data and/or commands are input to the processor 702.

The memory 704 stores a computer program 706 comprising computer program instructions (computer program code) that controls the operation of the apparatus 110, 120 when loaded into the processor 702. The computer program instructions, of the computer program 706, provide the logic and routines that enables the apparatus to perform the methods illustrated in Fig. 6 The processor 702 by reading the memory 704 is able to load and execute the computer program 706.

The apparatus 120 therefore comprises:
at least one processor 702; and
at least one memory 704 including computer program code;
the at least one memory 704 and the computer program code configured to, with the at least one processor 702, cause the apparatus 120 at least to perform:
receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and
wherein the identifiable output signal provides an indication of the presence of one or more chemicals detected by a plurality of genetically modified sensors coupled to the transducer array; and
using artificial intelligence to classify the identifiable output signal by determining any one or more of: an identity of a chemical, a class of chemicals, concentration of a chemical.

As illustrated in Fig. 7B, the computer program 706 may arrive at the apparatus 101 via any suitable delivery mechanism 710. The delivery mechanism 710 may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 706. The delivery mechanism may be a signal configured to reliably transfer the computer program 706. The apparatus 101 may propagate or transmit the computer program 706 as a computer data signal.

Computer program instructions for causing an apparatus 120 to perform at least the following or for performing at least the following:
receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and
wherein the identifiable output signal provides an indication of the presence of one or more chemicals detected by a plurality of genetically modified sensors coupled to the transducer array; and
using artificial intelligence to classify the identifiable output signal by determining any one or more of: an identity of a chemical, a class of chemicals, concentration of a chemical.

The computer program instructions may be comprised in a computer program, a non-transitory computer readable medium, a computer program product, a machine readable medium. In some but not necessarily all examples, the computer program instructions may be distributed over more than one computer program.

Although the memory 704 is illustrated as a single component/circuitry it may be implemented as one or more separate components/circuitry some or all of which may be integrated/removable and/or may provide permanent/semi-permanent/ dynamic/cached storage.

Although the processor 702 is illustrated as a single component/circuitry it may be implemented as one or more separate components/circuitry some or all of which may be integrated/removable. The processor 702 may be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

Where a structural feature has been described, it may be replaced by means for performing one or more of the functions of the structural feature whether that function or those functions are explicitly or implicitly described.

From the foregoing it will be appreciated that in some examples there is provided a system comprising:
an apparatus comprising:
   a plurality of genetically modified sensors comprising a plurality of subsets of sensors wherein the sensors are genetically modified so as to provide an output in response to one or more chemicals and wherein the plurality of genetically modified sensors are genetically modified in a non-correlated manner so that different subsets of sensors are configured to provide a different affinity to different chemicals; and
   a transducer array, comprising a plurality of pixels, wherein different subsets of the plurality of genetically modified sensors are coupled to different pixels so as to cause the pixels to provide outputs dependent upon the genetic modification of the subset of sensors coupled to the pixel, wherein
   detection of one or more chemicals by one or more of the genetically modified sensors causes an identifiable output signal to be provided by the transducer array and;
an artificial intelligence module comprising means for:
   receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals; and
   wherein the identifiable output signal provides an indication of the presence of one or more chemicals detected by a plurality of genetically modified sensors coupled to the transducer array; and
   using artificial intelligence to classify the identifiable output signal by determining any one or more of: an identity of a chemical, a class of chemicals, concentration of a chemical.

In some but not necessarily all examples, the apparatus 101 is configured to communicate data from the apparatus 101 with or without local storage of the data in a memory at the apparatus 101 and with or without local processing of the data by circuitry or processors at the apparatus 101. The data may be the identifiable output signal 111 or data produced by the processing the identifiable output signal 111.

The data may be stored in processed or unprocessed format remotely at one or more devices. The data may be stored in the Cloud.

The data may be processed remotely at one or more devices. The data may be partially processed locally and partially processed remotely at one or more devices.

The data may be communicated to the remote devices wirelessly via short range radio communications such as Wi-Fi or Bluetooth, for example, or over long range cellular radio links. The apparatus may comprise a communications interface such as, for example, a radio transceiver for communication of data.

The apparatus 101 and/or the artificial intelligence module may be part of the Internet of Things forming part of a larger, distributed network.

The processing of the data, whether local or remote, may be for the purpose of health monitoring, data aggregation, patient monitoring, vital signs monitoring or other purposes.

The processing of the data, whether local or remote, may involve artificial intelligence or machine learning algorithms. The data may, for example, be used as learning input to train a machine learning network or may be used as a query input to a machine learning network, which provides a response. The machine learning network may for example use linear regression, logistic regression, vector support machines or an acyclic machine learning network such as a single or multi hidden layer neural network.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example. Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising:
a plurality of genetically modified sensors comprising a plurality of subsets of sensors wherein the sensors are genetically modified so as to provide an output in response to one or more chemicals and wherein the plurality of genetically modified sensors are genetically modified in a non-correlated manner so that different subsets of sensors are configured to provide a different affinity to different chemicals; and
a transducer array, comprising a plurality of pixels, wherein different subsets of the plurality of genetically modified sensors are coupled to different pixels so as to cause the pixels to provide outputs dependent upon the genetic modification of the subset of sensors coupled to the pixel, wherein
detection of one or more chemicals by one or more of the genetically modified sensors causes an identifiable output signal to be provided by the transducer array.

2. An apparatus as claimed in claim 1 wherein the genetically modified sensors within a subset are homogenous.

3. An apparatus as claimed in any preceding claim wherein the identifiable output signal from the transducer array comprises a combination of outputs from a plurality of different pixels.

4. An apparatus as claimed in any preceding claim wherein the genetically modified sensors are adsorbed to the pixels of the transducer array.

5. An apparatus as claimed in any preceding claim wherein the transducer array comprises at least one of mass sensitive transducers, light sensitive transducers, electrically sensitive transducers, and/or heat sensitive transducers.

6. An apparatus as claimed in any preceding claim wherein the genetically modified sensors comprise at least one of viral particles, desiccation tolerant cells, synthetic peptides, randomized DNA, proteins, and/or receptors.

7. An apparatus as claimed in any preceding claim wherein the genetically modified sensors are genetically modified to sense chemicals in at least one of, gas phase, and/or a liquid phase.

8. An apparatus as claimed in any preceding claim wherein the genetically modified sensors are genetically modified in a random or pseudo random manner.

9. An apparatus as claimed in any preceding claim further comprising means for providing the identifiable output signal of the transducer array to an artificial intelligence module to enable the artificial intelligence module to classify the identifiable output signal.

10. An apparatus as claimed in claim 9 wherein classifying the identifiable output signal comprises one or more of: determining an identity of a chemical, determining a class of chemicals, determining concentration of a chemical.

11. An apparatus as claimed in any of claims 9 to 10 wherein the artificial intelligence module is configured to use a pattern recognition algorithm to classify the identifiable output signal.

12. An apparatus comprising means for:
receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals;
wherein the identifiable output signal provides an indication of the presence of one or more chemicals detected by a plurality of genetically modified sensors coupled to the transducer array; and
using artificial intelligence to classify the identifiable output signal by determining any one or more of: an identity of a chemical, a class of chemicals, concentration of a chemical.

13. An apparatus as claimed in claim 12 wherein training data obtained by exposing the transducer array and plurality of genetically modified sensors to one or more known chemicals is used to classify the identifiable output signal from the transducer array.

14. An apparatus as claimed in any preceding claim wherein a pattern recognition algorithm is used to classify the identifiable output signal from the transducer array.

15. A method comprising:
receiving an identifiable output signal from a transducer array, wherein the transducer array comprises a plurality of pixels with a different subset of genetically modified sensors coupled to different pixels within the transducer array where the sensors have been genetically modified in a non-correlated manner so that different subsets of sensors are configured to provide a different affinity to different chemicals;
wherein the identifiable output signal provides an indication of the presence of one or more chemicals detected by a plurality of genetically modified sensors coupled to the transducer array; and
using artificial intelligence to classify the identifiable output signal by determining any one or more of: an identity of a chemical, a class of chemicals, concentration of a chemical.
